(19) Europäisches Patentamt
European Patent Office
Office européen des brevets

(11) Veröffentlichungsnummer: **0 198 797**
**B1**

(12) # EUROPÄISCHE PATENTSCHRIFT

(45) Veröffentlichungstag der Patentschrift: **31.01.90**

(51) Int. Cl.⁴: **C07D 317/46,** A01N 43/30

(21) Anmeldenummer: **86810145.2**

(22) Anmeldetag: **26.03.86**

(54) **Schädlingsbekämpfungsmittel.**

(30) Priorität: **03.04.85 CH 1434/85**

(43) Veröffentlichungstag der Anmeldung:
**22.10.86 Patentblatt 86/43**

(45) Bekanntmachung des Hinweises auf die Patenterteilung:
**31.01.90 Patentblatt 90/5**

(84) Benannte Vertragsstaaten:
**AT BE CH DE FR IT LI LU NL SE**

(56) Entgegenhaltungen:
**EP-A- 0 005 756**

(73) Patentinhaber: **CIBA-GEIGY AG, Klybeckstrasse 141, CH-4002 Basel(CH)**

(72) Erfinder: **Ehrenfreund, Josef, Dr., Amselstrasse 11, CH-4123 Allschwil(CH)**

EP 0 198 797 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die vorliegende Erfindung betrifft die neue Anilino-benzodioxol-Verbindung der nachfolgenden Formel I. Die Erfindung betrifft ferner die Herstellung dieser Verbindung und die Mittel, die diese Verbindung als aktive Komponente enthalten, sowie die Verwendung dieses Wirkstoffs oder der genannten Mittel zur Bekämpfung von Schädlingen, vorzugsweise pflanzenschädigenden Pilzen oder Bakterien, ferner von Insekten und Akariden.

Die erfindungsgemässe Verbindung entspricht der Formel I

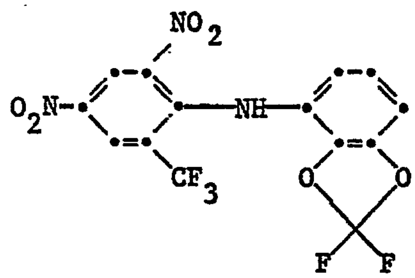

(I).

Die Herstellung dieser Verbindung erfolgt erfindungsgemäss durch Umsetzung der Verbindung der Formel II

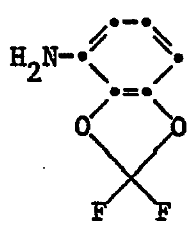

(II)

mit der Verbindung der Formel III

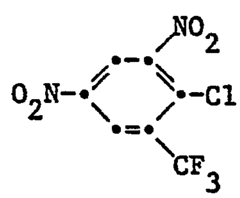

(III)

in Gegenwart eine säurebindenden Mittels in einem inerten Lösungs-oder Verdünnungsmittel

Geeignete Lösungs- oder Verdünnungsmittel sind beispielsweise aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Dioxan, Tetrahydrofuran; Nitrile wie Acetronitril, Propionitril; N,N-dialkylierte Amide wie Dimethylformamid; Dimethylsulfoxid; Ketone wie Aceton, Diethylketon, Methylethylketon und Gemische solcher Lösungsmittel untereinander. Bevorzugt sind Dimethylformamid, Tetrahydrofuran oder Dioxan. In manchen Fällen ist es vorteilhaft, in wässrigen Suspensionen zu arbeiten.

Als säurebindende Mittel eignen sich anorganische Basen, z.B. Oxide, Hydroxide, Carbonate oder Hydrogencarbonate von Alkali- oder Erdalkalimetallen sowie Alkalihydride oder Alkaliacetate, ferner organische Basen, z.B. tertiäre Amine wie Trialkylamine (Trimethylamin, Triethylamin usw.), Pyridin oder Pyridinbasen (4-Dimethylaminopyridin, 4-Pyrrolidylaminopyridin).Bevorzugt sind Alkalihydroxide, Alkalihydrogencarbonat oder Alkalihydrid.

Die Reaktionstemperatur ist je nach den Reaktionsbedingungen variabel. Sie liegt im allgemeinen zwischen -25° und 150°C, vorzugsweise zwischen +10° und 120°C.

Normalerweise werden die Reaktionspartner in äquimolaren Mengen eingesetzt.

Die als Ausgangsmaterial zur Herstellung der erfindungsgemässen Verbindung der Formel I verwendete Verbindung der Formel II ist neu. Sie stellt ein wertvolles Zwischenprodukt für die Synthese biozider Wirkstoffe dar und ist ein Bestandteil der vorliegenden Erfindung. Ihre Herstellung erfolgt z.B.

durch Hofmann-Abbau, d.h. durch Umsetzung von 2,2-Difluor-4-carbamoyl-1,3-benzodioxol mit Brom in Gegenwart von wässerigem Alkalihydroxid.

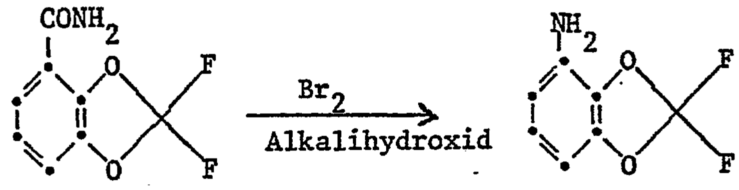

(II)

Die Durchführung dieses Verfahrens verläuft analog der Methode von L.M. Yagupolskii et al Zhur.Obshch.Khym. 31, 628 (1981).
Die Vorstufen zur Herstellung der Verbindung der Formel II werden ausgehend von 4-Carboxy-1,3-benzodioxol (nach W.H. Perkin und V.M. Trikojus, J. Chem.Soc. 1926, 2925) wie folgt synthetisiert:

1)

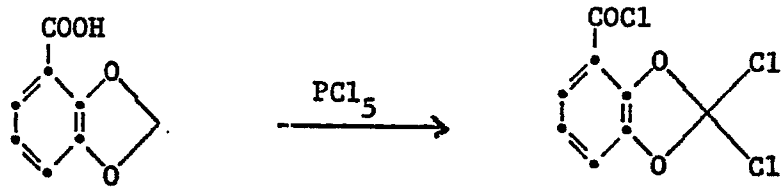


2)

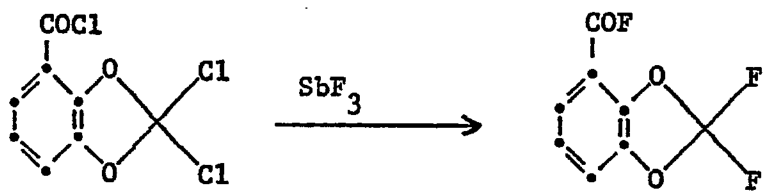


3)

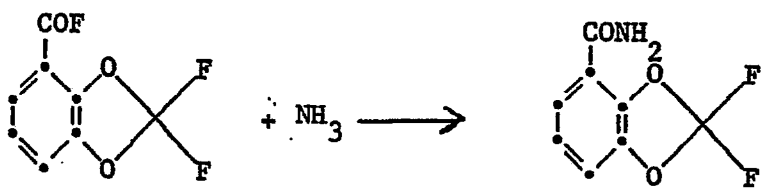


Es sind bereits Diarylamine bekannt, die zur Verwendung als Fungizide, Insektizide und Akarizide vorgeschlagen worden sind (vgl. DE-OS 28 23 168). Ihre Wirksamkeit hat sich jedoch nicht im gewünschten Masse als voll befriedigend erwiesen. So ist z.B. aus der vorgenannten DE-OS folgende Verbindung A bekannt:

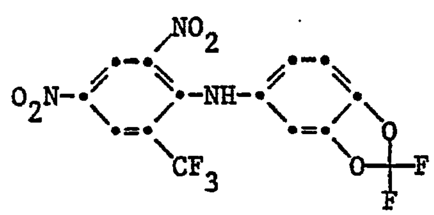
(A).

Es wurde nun überraschend festgestellt, dass die erfindungsgemässe Verbindung der Formel I ein sehr vorteilhaftes, die praktischen Bedürfnisse gut befriedigendes biozides Wirkungsspektrum gegen Pilze, Bakterien, Insekten und Vertreter der Ordnung Acarina, insbesondere gegen phytopathogene Pilze und Bakterien,aufweist. Sie besitzt sehr vorteilhafte kurative, systemische und insbesondere präventive Eigenschaften und lässt sich zum Schutz von zahlreichen Kulturpflanzen einsetzen. Mit dem Wirkstoff der Formel I können an Pflanzen oder an Pflanzenteilen (Früchte, Blüten, Laubwerk, Stengel. Knollen, Wurzeln) von unterschiedlichen Nutzkulturen die auftretenden Schädlinge eingedämmt oder

vernichtet werden, wobei auch später zuwachsende Pflanzenteile vor phytopathogenen Mikroorganismen und Insekten verschont bleiben. Ferner können Acarina erfolgreich mit dem Wirkstoff der Formel I bekämpft werden.

Als Mikrobizid ist der Wirkstoff der Formel I z.B. gegen die den folgenden Klassen angehörenden phytopathogenen Pilze wirksam: Fungi imperfecti (z.B. Botrytis, Helminthosporium, Fusarium, Septoria, Cercospora und Alternaria); Basidiomyceten (z.B. die Gattungen Hemileia, Rhizocotonia, Puccinia); sowie gegen die der Klasse Phycomycetes angehörenden Oomycetes (z.B. Plasmopara viticola); insbesondere gegen die Klasse der Ascomyceten (z.B. Venturia, Podosphaera, Erysiphe, Monilinia, Uncinula). Ueberdies wirkt die Verbindung der Formel I systemisch. Sie kann ferner als Beizmittel zur Behandlung von von Saatgut (Früchte, Knollen, Körner) und Pflanzenstecklingen zum Schutz von Pilzinfektionen sowie gegen im Erdboden auftretende phytopathogene Pilze eingesetzt werden.

Als Insektizid kann die Verbindung der Formel I auch zur Bekämpfung von Insekten der Ordnung: Lepidoptera, Coleoptera, Homoptera, Heteroptera, Diptera, Thysanoptera, Orthoptera, Blattaria, Anoplura, Siphonaptera, Mallophaga, Thysanura, Isoptera, Psocoptera und Hymenoptera eingesetzt werden.

Die Verbindung der Formel I ist weiterhin geeignet zur Bekämpfung von Vertretern der Ordnung Acarina, z.B. der Familien Ioxididae, Argasidae, Tetranychidae und Dermanyssidae. Die Verbindung der Formel I kann mit Erfolg zur Bekämpfung von phytopathogenen Milben, z.B. der Familie Tetranychidae und Phytoptipalpidae (Spinnmilben), Tarsonemidae (Fadenfussmilben) und Eriophydiae (Gallmilben), eingesetzt werden.

Neben ihrer Wirkung gegenüber Mücken und Fliegen, wie z.B. Aedes aegypti und Musca domestica, kann die Verbindung der Formel I auch zur Bekämpfung von pflanzenschädigenden Frassinsekten in Zier- und Nutzpflanzungen, insbesondere in Baumwollkulturen (z.B. gegen Spodoptera littoralis und Heliothis virescens), sowie in Getreide-, Obst- und Gemüsekulturen (z.B. gegen Laspeyresia pomonella, Leptinotarsa decemlineata und Epilachna varivestis) eingesetzt werden. Die Verbindung der Formel I zeichnet sich auch durch eine gute Wirkung gegen larvale Entwicklungsstadien und Nymphen, insbesondere von fressenden Schadinsekten, aus.

Die Verbindung der Formel I kann ferner zur Bekämpfung von ektoparasitären Insekten und Acariden an Haus- und Nutztieren verwendet werden, z.B. durch Tier-, Stall- und Weidebehandlung.

Die Wirkung der erfindungsgemässen Verbindung bzw. der sie enthaltenden Mittel lässt sich durch Zusatz von anderen Insektiziden und/oder Akariziden wesentlich verbreitern und an gegebene Umstände anpassen.

Die Erfindung betrifft somit auch Schädlingsbekämpfungsmittel (Mikrobizide, Insektizide, Akarizide) sowie deren Verwendung auf dem Agrarsektor oder verwandten Gebieten.

Darüber hinaus schliesst die vorliegende Erfindung auch die Herstellung dieser Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das in der Applikation der Verbindung der Formel I bzw. der neuen Mittel besteht.

Als Zielkulturen für die hierin offenbarten Einsatz gelten im Rahmen dieser Erfindung beispielsweise folgende Pflanzenarten: Getreide (Weizen, Gerste, Roggen, Hafer, Reis, Sorghum und Verwandte); Rüben (Zucker- und Futterüben); Kern-, Stein- und Beerenobst (Aepfel, Birnen, Pflaumen, Pfirsiche, Mandeln, Kirschen, Erd-, Him- und Brombeeren); Hülsenfrüchte (Bohnen, Linsen, Erbsen, Soja); Oelkulturen (Raps, Senf, Mohn, Oliven, Sonnenblumen, Kokos, Rizinus, Kakao, Erdnüsse); Gurkengewächse (Kürbis, Gurken, Melonen); Fasergewächse (Baumwolle, Flachs, Hanf, Jute); Citrusfrüchte (Orangen, Zitronen, Grapefruit, Mandarinen); Gemüsesorten (Spinat, Kopfsalat, Spargel, Kohlarten, Möhren, Zwiebeln, Tomaten, Kartoffeln, Paprika); Lorbeergewächse (Avocado, Cinnamonum, Kampfer) oder Pflanzen wie Mais, Tabak, Nüsse, Kaffee, Zuckerrohr, Tee, Weinreben, Hopfen, Bananen-und Naturkautschukgewächse sowie Zierpflanzen (Compositen).

Der Wirkstoff der Formel I wird üblicherweise in Form von Zusammensetzungen mit Hilfsingredienzien, wie Trägerstoffen und Verdünnungsmitteln, verwendet und kann gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen des Wirkstoffes der Formel I bzw. eines agrochemischen Mittels, das diesen Wirkstoff enthält, ist das Aufbringen auf das Blattwerk (Blattapplikation). Anzahl der Applikationen und Aufwandmenge richten sich dabei nach dem Befallsdruck des entsprechenden Parasiten (z.B. Pilzsorte). Der Wirkstoff der Formel I kann aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt, z.B. in

Form von Granulat (Bodenapplikation). Die Verbindung der Formel I kann aber auch auf Samenkörner aufgebracht werden (Coating, Beizung), indem man die Körner entweder mit einer flüssigen Zubereitung des Wirkstoffs tränkt oder sie mit einer festen Zubereitung beschichtet. Darüber hinaus sind in besonderen Fällen weitere Applikationsarten möglich, so z.B. die gezielte Behandlung der Pflanzenstengel oder der Knospen.

Die Verbindung der Formel I wird dabei in unveränderter Form oder vorzugsweise zusammen mit den in der Formulierungstechnik üblichen Hilfsmitteln eingesetzt und wird daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt und angepasst. Günstige Aufwandmengen liegen im Allgemeinen bei 50 g bis 5 kg Aktivsubstanz (AS) je ha; bevorzugt 100 g bis 2 kg AS/ha, insbesondere bei 200 g bis 600 g AS/ha.

Die Formulierungen d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Also gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinerte Pflanzenrückstände verwendet werden. Besonders vorteilhafte, applikationsfördernde Zuschlagstoffe, die zu einer starken Reduktion der Aufwandmenge führen können, sind ferner natürliche (tierische oder pflanzliche) oder synthetische Phospholipide aus der Reihe der Kephaline und Lecithine, wie z.B. Phosphatidylethanolamin, Phosphatidylserin, Phosphatidylcholin, Sphingomyelin, Phosphatidylinosit, Phosphatidylglycerin, Lysolecithin, Plasmalogene oder Cardiolipin, die man beispielsweise aus tierischen oder pflanzlichen Zellen, insbesondere aus Hirn, Herz, Leber, Eidotter oder Sojabohnen gewinnen kann. Verwendbare Handelsmischungen sind z.B. Phosphatidylcholin-Mischungen. Synthetische Phospholipide sind z.B. Dioctanoylphosphatidylcholin und Dipalmitoylphosphatidylcholin.

Als oberflächenaktive Verbindungen kommen je nach Art des zu formulierenden Wirkstoffes der Formel I nichtionogene, Kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäure-methyllaurinsalze zu erwähnen.

Häufiger werden jedoch sog. synthetische Tenside verwendet, insbesondere Fettsulfonate, Fettsulfate, sulfonierte Benzimidazolderivate oder Alkylsulfonate.

Die Fettsulfonate oder -sulfate liegen in der Regel als Alkali-, Erdalkali- oder gegebenenfalls substituierte Ammoniumsalze vor und weisen einen Alkalirest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxyd-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxyd-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3

bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 g Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxidaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitantrioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Ridgewood New Jersey, 1981;

Sisley and Wood, "Encyclopedia of Surface Active Agents", Chemical Publishing Co., Inc. New York, 1980;

Helmut Stache "Tensid-Taschenbuch" Carl Hanser-Verlag München/Wien 1981.

Die agrochemischen Zubereitungen enthalten in der Regel 0,1 bis 99%, insbesondere 0,1 bis 95% Wirkstoff der Formel I, 99,9 bis 1%, insbesondere 99,8 bis 5% eines festen oder flüssigen Zusatzstoffes und 0 bis 25%, insbesondere 0,1 bis 25% eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige agrochemische Mittel sind Bestandteil der vorliegenden Erfindung.

Die nachfolgenden Beispiele dienen zur näheren Erläuterung der Erfindung, ohne dieselbe einzuschränken.

1. <u>Herstellungsbeispiele</u>

Beispiel 1.1: Herstellung von 2,2-Difluor-4-[2',4'-dinitro-6'-(trifluormethyl)-anilino]-1,3-benzodioxol

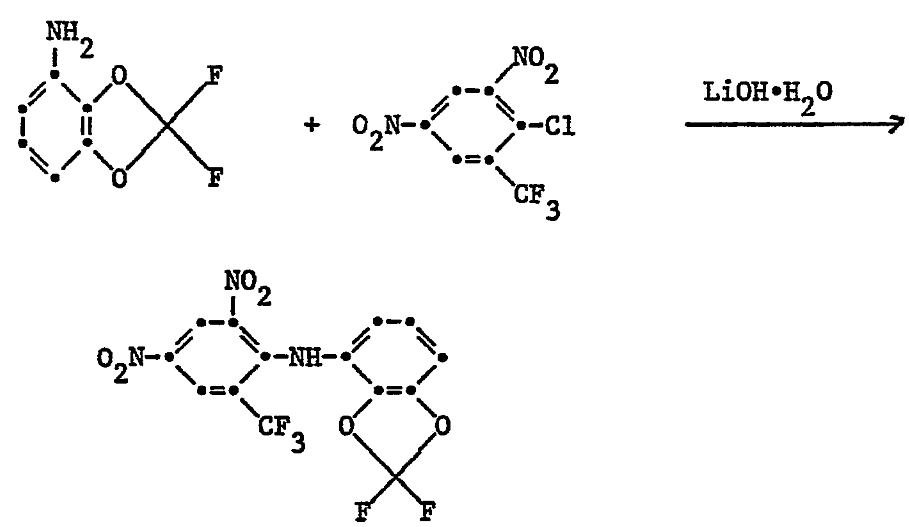


In eine Lösung von 24,5 g 2,2-Difluor-4-amino-1,3-benzodioxol in 70 ml Dimethylsulfoxid werden bei 0°C 20,8 g $LiOH \cdot H_2O$ gegeben. Anschliessend wird das Gemisch 20 Minuten gerührt. Nachfolgend werden 38,3 g 2,4-Dinitro-6-trifluormethyl-chlorbenzol gelöst in 70 ml Dimethylsulfoxid hinzugefügt, das gebildete Reaktionsgemisch bei 0°C 20 Minuten gerührt und dann auf Raumtemperatur erwärmt. Nach 5 Stunden wird das Gemisch in 350 ml Wasser gegossen, mit verdünnter Salzsäure angesäuert und mehrfach

mit Diethylether extrahiert. Die vereinigten Etherphasen werden je dreimal mit 5%iger Sodalösung und dann mit 5%iger Salzsäure gewaschen, über Natriumsulfat getrocknet und das Lösungsmittel unter Vakuum abgedampft. Den Rückstand bilden 51,6 g orangebraune Kristalle, welche in Toluol umkristallisiert werden.
Ausbeute: 41,6 g; Schmelzpunkt: 144-146°C.

Beispiel 1.2:Herstellung von 2,2-Difluor-4-amino-1,3-benzodioxol

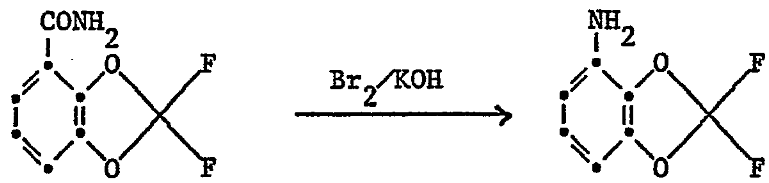


In eine Lösung von 120 g Kaliumhydroxid in 780 ml Wasser werden bei 0°C 52,5 Brom eingetropft. Anschliessend werden 50,8 g 2,2-Difluor-4-carbomoyl-1,3-benzodioxol hinzugefügt und das Reaktionsgemisch 30 Minuten bei Raumtemperatur gerührt und weitere 45 Minuten auf 80°C erwärmt. Dann wird die Reaktionslösung auf 400 ml Wasser verdünnt und mit Wasserdampf destilliert. Das Destillat wird mit Diethylether extrahiert und über Natriumsulfat getrocknet. Nach Verdampfen des Lösungsmittels erhält man 38,1 g der Titelverbindung als gelbliches Oel mit einem Brechungsindex von $n^{21}_D$ 1.4955.

Beispiel 1.3: Herstellung von 2,2-Dichlor-4-carbonylchlorid-1,3-benzodioxol

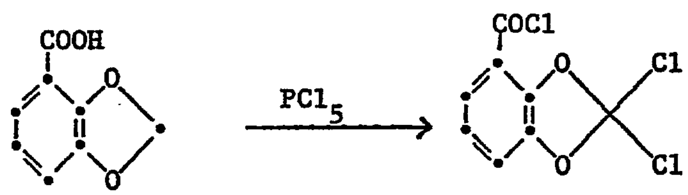


40 g 4-Carboxy-1,3-benzodioxol werden mit 150 g Phosphorpentachlorid gemischt und auf 70°C erwärmt. Nach 3,5 Stunden wird die Temperatur auf 90°C erhöht und 12 Stunden bei dieser Temperatur belassen. Anschliessend werden die flüchtigen Anteile bei 110°C und 2000 Pa abdestilliert und der Rückstand unter Hochvakuum abdestilliert. Es werden 45 g der Titelverbindung als leicht gelblicher Feststoff erhalten.

Beispiel 1.4: Herstellung von 2,2-Difluor-4-carbonylfluorid-1,3-benzodioxol

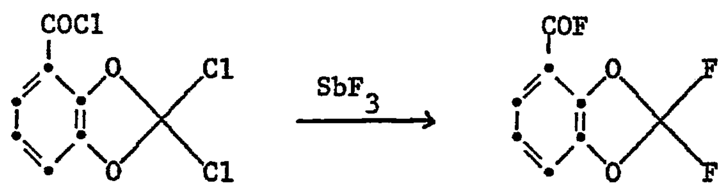


Zu 59,4 g 2,2-Dichlor-4-carbonylchlorid-1,3-benzodioxol werden in einer Destillationsapparatur 55,7 g $SbF_3$ gegeben, wobei sofort eine Reaktion eintritt. Zur Aufrechterhaltung der Reaktion wird die Temperatur bei gleichzeitiger allmählicher Evakuierung langsam erhöht. Bei 105-117°C und 6666 Pa destillieren 59,5 g eines roten Oels über. Das Destillat wird in 100 ml Diethylether aufgenommen, zweimal mit je 150 ml halbkonzentrierter Salzsäure und anschliessend mit Wasser gewaschen. Nach Trocknen der Etherlösung über Natriumsulfat wird das Lösungsmittel verdampft. Es werden 42,0 g der Titelverbindung erhalten.

Beispiel 1.5: Herstellung von 2,2-Difluor-4-carbamoyl-1,3-Benzodioxol

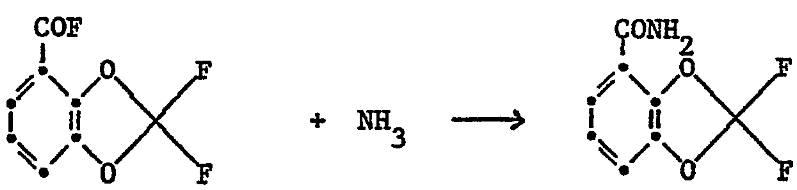

63,6 g 2,2-Difluor-4-carbonylfluorid-1,3-benzodioxol werden unter Eiskühlung und Rühren zu 120 ml konz. Ammoniaklösung (25%ig) zugetropft. Im Laufe von einigen Stunden bildet sich ein Kristallbrei, der anschliessend abfiltriert, nacheinander mit verdünnter Ammoniaklösung und dann mit Wasser gewaschen wird. Es werden 51 g 2,2-Difluor-4-carbamoyl-1,3-benzodioxol mit einem Schmelzpunkt von 129,5-131°C erhalten.

2. Formulierungsbeispiele für den Wirkstoff der Formel I (% = Gewichtsprozent)

| 2.1 Emulsions-Konzentrate | a) | b) | c) |
|---|---|---|---|
| Wirkstoff | 25% | 40% | 50% |
| Ca-Dodecylbenzolsulfonat | 5% | 8% | 6% |
| Ricinusöl-polyethylenglykolether (36 Mol Ethylenoxid) | 5% | – | – |
| Tributylphenoyl-polyethylenglykolether (30 Mol Ethylenoxid) | – | 12% | 4% |
| Cyclohexanon | – | 15% | 20% |
| Xylolgemisch | 65% | 25% | 20% |

Aus solchen Konzentraten können durch Verdünnen mit Wasser Emulsionen jeder gewünschten Konzentration hergestellt werden.

| 2.2 Lösungen | a) | b) | c) | d) |
|---|---|---|---|---|
| Wirkstoff | 80% | 10% | 5% | 95% |
| Ethylenglykol-monomethyl-ether | 20% | – | – | – |
| Polyethylenglykol MG 400 | – | 70% | – | – |
| N-Methyl-2-pyrrolidon | – | 20% | – | – |
| Epoxydiertes Kokosnussöl | – | – | 1% | 5% |
| Benzin (Siedegrenzen 160–190 °C) | – | – | 94% | – |
| (MG = Molekulargewicht) | | | | |

Die Lösungen sind zur Anwendung in Form kleinster Tropfen geeignet.

| 2.3 Granulate | a) | b) |
|---|---|---|
| Wirkstoff | 5% | 10% |
| Kaolin | 94% | – |
| Hochdisperse Kieselsäure | 1% | – |
| Attapulgit | – | 90% |

Der Wirkstoff wird in Methylenchlorid gelöst, auf den Träger aufgesprüht und das Lösungsmittel anschliessend im Vakuum abgedampft.

| 2.4 Stäubemittel | a) | b) |
|---|---|---|
| Wirkstoff | 2% | 5% |
| Hochdisperse Kieselsäure | 1% | 5% |
| Talkum | 97% | – |
| Kaolin | – | 90% |

Durch inniges Vermischen auf Trägerstoffe mit dem Wirkstoff erhält man gebrauchsfertige Stäubemittel.

| 2.5 Spritzpulver | a) | b) | c) |
|---|---|---|---|
| Wirkstoff | 25% | 50% | 75% |
| Na-Ligninsulfonat | 5% | 5% | – |
| Na-Laurylsulfat | 3% | – | 5% |
| Na-Diisobutylnaphthalinsulfonat | – | 6% | 10% |
| Octylphenolpolyethylenglykolether (7–8 Mol Ethylenoxid) | – | 2% | – |
| Hochdisperse Kieselsäure | 5% | 10% | 10% |
| Kaolin | 62% | 27% | – |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 2.6 Emulsions-Konzentrat | |
|---|---|
| Wirkstoff | 10% |
| Octylphenolpolyethylenglykolether (4–5 Mol Ethylenoxid) | 3% |
| Ca-Dodecylbenzolsulfonat | 3% |
| Ricinusölpolyglykolether (35 Mol Ethylenoxid) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| 2.7 Stäubemittel | a) | b) |
|---|---|---|
| Wirkstoff | 5% | 8% |
| Talkum | 95% | – |
| Kaolin | – | 92% |

Man erhält anwendungsfertige Stäubemittel, indem der Wirkstoff mit den Träger vermischt auf einer geeigneten Mühle vermahlen wird.

| 2.8 Extruder Granulat | |
|---|---|
| Wirkstoff | 10% |
| N-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschliessend im Luftstrom getrocknet.

| 2.9 Umhüllungs-Granulat | |
|---|---|
| Wirkstoff | 3% |
| Polyethylenglykol (MG 200) | 3% |
| Kaolin | 94% |

(MG = Molekulargewicht)

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

| 2.10 Suspensions-Konzentrat | |
|---|---|
| Wirkstoff | 40% |
| Ethylenglykol | 10% |
| Nonylphenolpolyethylenglykolether (15 Mol Ethylenoxid) | 6% |
| N-Ligninsulfonat | 10% |
| Carboxymethylcellulose | 1% |
| 37%ige wässrige Formaldehyd-Lösung | 0,2% |
| Silikonöl in Form einer 75%igen wässrigen Emulsion | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

3. Biologische Beispiele

In den nachfolgenden Versuchen (Beispiele 3.1-3.5) wurden die erfindungsgemässe Verbindung der Formel I

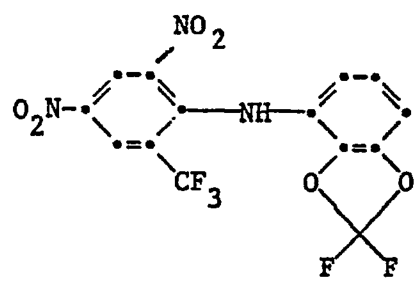

(I)

und die Verbindung des Beispiels 1 aus der Deutschen Offenlegungsschrift Nr. 28 23 168

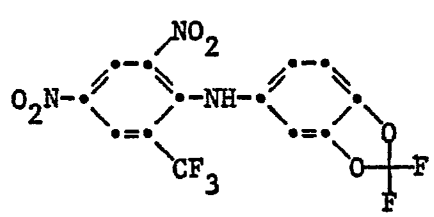

(A)

einem Vergleich ihrer fungiziden Aktivitäten unterzogen.

Beispiel 3.1: Wirkung gegen Plasmopara viticola auf Reben

Residual-protektive Wirkung

Im 4-5 Blattstadium werden Rebensämlinge mit einer aus Spritzpulver des Wirkstoffes hergestellten Spritzbrühe (0,006% Aktivsubstanz) besprüht. Nach 24 Stunden werden die behandelten Pflanzen mit einer Sporangiensuspension des Pilzes infiziert. Nach einer Inkubation während 6 Tagen bei 95-100% relativer Luftfeuchtigkeit und 20°C wird der Pilzbefall beurteilt.

Beispiel 3.2: Wirkung gegen Piricularia oryzae auf Reispflanzen

Residual-protektive Wirkung

Reispflanzen werden nach zweiwöchiger Anzucht mit einer aus Spritzpulver des Wirkstoffes hergestellten Spritzbrühe (0,006% Aktivsubstanz) besprüht. Nach 48 Stunden werden die behandelten Pflanzen mit einer Konidiensuspension des Pilzes infiziert. Nach 5 Tagen Inkubation bei 95-100% relativer Luftfeuchtigkeit und 24°C wird der Pilzbefall beurteilt.

Beispiel 3.3: Wirkung gegen Cercospora arachidicola auf Erdnusspflanzen

Residual-protektive Wirkung

10-15 cm hohe Erdnusspflanzen werden mit einer aus Spritzpulver der Wirksubstanz hergestellten Spritzbrühe (0,006% Aktivsubstanz) besprüht und 48 Stunden später mit einer Konidiensuspension des Pilzes infiziert. Die infizierten Pflanzen werden während 72 Stunden bei ca. 21°C und hoher Luftfeuchtigkeit inkubiert und anschliessend bis zum Auftreten der typischen Blattflecken in einem Gewächshaus aufgestellt. Die Beurteilung der fungiziden Wirkung erfolgt 12 Tage nach der Infektion basierend auf Anzahl und Grösse der auftretenden Flecken.

Beispiel 3.4: Wirkung gegen Venturia inaequalis auf Apfeltrieben

Residual-protektive Wirkung

Apfelstecklinge mit 10-20 cm langen Frischtrieben werden mit einer aus Spritzpulver des Wirkstoffes hergestellten Spritzbrühe (0,006% Aktivsubstanz) besprüht. Nach 24 Stunden werden die behandelten Pflanzen mit einer Konidiensuspension des Pilzes infiziert. Die Pflanzen werden dann während 10 weiteren Tagen in einem Gewächshaus bei 20-24°C aufgestellt. Der Schorfbefall wird 15 Tage nach der Infektion beurteilt.

Beispiel 3.5: Wirkung gegen Botrytis cinerea an Apfelfrüchten

Residual-protektive Wirkung

Künstlich verletzte Aepfel werden behandelt, indem eine aus Spritzpulver der Wirksubstanz hergestellte Spritzbrühe (0,006% Aktivsubstanz) auf die Verletzungsstellen aufgetropft wird. Die behandelten Früchte werden anschliessend mit einer Sporensuspension des Pilzes inokuliert und während einer Woche bei hoher Luftfeuchtigkeit und ca. 20°C inkubiert. Bei der Auswertung werden die angefaulten Verletzungsstellen gezählt und daraus die fungizide Wirkung der Testsubstanz abgeleitet.

Ergebnis:

| Wirkstoff | Testorganismen (FUNGI) | | | | |
|---|---|---|---|---|---|
| | Plasmopara viticola | Piricularia oryzae | Cercospora arachidicola | Venturia inaequalis | Botrytis cinerea |
| I | 0 | 1 | 0 | 1 | 0 |
| A | 3 | 3 | 3 | 2 | 3 |

Bewertung:

Die Benotung bezieht sich auf den prozentualen Pilzbefall im Vergleich mit infizierten, jedoch unbehandelten Kontrollpflanzen.

| Note | %-Befall |
|---|---|
| 0 | 0–5 |
| 1 | 5–20 |
| 2 | 20–50 |
| 3 | > 50 (inaktiv) |

Beispiel 3.6: Wirkung gegen OP-resistente Spinnmilben (Tetranychus cinnabarinus)

Bohnenpflanzen im Primärblattstadium werden 24 Stunden vor der Behandlung mit einer Mischpopulation von Tetranychus besiedelt. Anschliessend werden die Pflanzen mit der Testlösung, enthaltend 200 ppm der Prüfverbindung behandelt. Die Auswertung auf Mortalität der einzelnen Stadien erfolgt nach 7 Tagen. Die erfindungsgemässe Verbindung zeigt bei diesem Test volle Wirkung (keine lebenden Individuen).

## Patentansprüche

1. 2,2-Difluor-4-(2',4'-dinitro-6'-trifluormethyl-anilino)-1,3-benzodioxol der Formel I

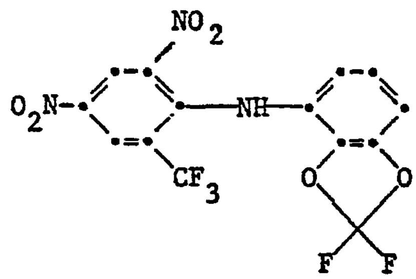

(I).

2. Verfahren zur Herstellung der Verbindung der Formel I, dadurch gekennzeichnet, dass man die Verbindung der Formel II

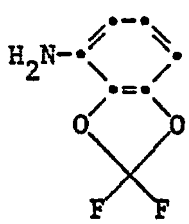

(II)

mit der Verbindung der Formel III

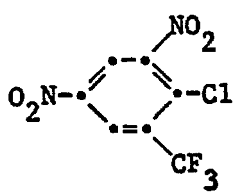

(III)

in Gegenwart eine säurebindenden Mittels in einem inerten Lösungsmittel umsetzt.

3. Mittel zur Bekämpfung oder Verhütung eines Befalls durch Schädlinge, dadurch gekennzeichnet, dass es als aktive Komponente die Verbindung der Formel I gemäss Anspruch 1 enthält.

4. Mittel nach Anspruch 3, dadurch gekennzeichnet, dass es 0,1 bis 99% des Wirkstoffs der Formel I, 99,9 bis 1% eines festen oder flüssigen Zusatzstoffes und 0 bis 25% eines Tensides enthält.

5. Mittel nach Anspruch 4, dadurch gekennzeichnet, dass es 0,1 bis 95% des Wirkstoffs der Formel I, 99,8 bis 5% eines festen oder flüssigen Zusatzstoffes und 0,1 bis 25% eines Tensids enthält.

6. Verfahren zur Herstellung eines agrochemischen Mittels gemäss Anspruch 3, dadurch gekennzeichnet, dass man die in Anspruch 1 definierte Verbindung der Formel I mit geeigneten festen oder flüssigen Zusatzstoffen und Tensiden innig vermischt.

7. Verfahren zur Bekämpfung oder Verhütung eines Befalls von Kulturpflanzen durch phytopathogene Schädlinge, dadurch gekennzeichnet, dass man die in Anspruch 1 definierte Verbindung der Formel I auf die Pflanze oder deren Standort appliziert.

8. Verwendung der Verbindung der Formel I gemäss Anspruch 1 zur Bekämpfung und/oder präventiven Verhütung eines Befalls von Mikroorganismen.

9. Verwendung gemäss Anspruch 8, dadurch gekennzeichnet, dass es sich bei den Mikroorganismen um phytopathogene Pilze handelt.

10. Verwendung gemäss Anspruch 19 gegen Pilze aus der Klasse Ascomycetes, Basidiomycetes oder Fungi imperfecti.

11. Verwendung der Verbindung der Formel I zur Bekämpfung von Insekten.

12. Verwendung der Verbindung der Formel I zur Bekämpfung von Akarina.

13. Verbindung der Formel II

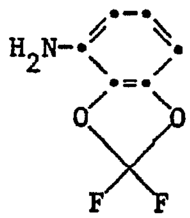

(II) .

14. Verfahren zur Herstellung der Verbindung der Formel II, dadurch gekennzeichnet, dass man die Verbindung der Formel

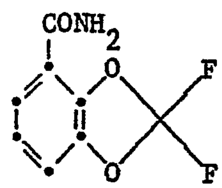

mit Brom in Gegenwart von wässrigem Alkalihydroxid dem Hofmann-Abbau unterwirft.

**Claims**

1. 2,2-Difluoro-4-(2', 4'-dinitro-6'-trifluoromethylanilino)-1,3-benzodioxole of formula I

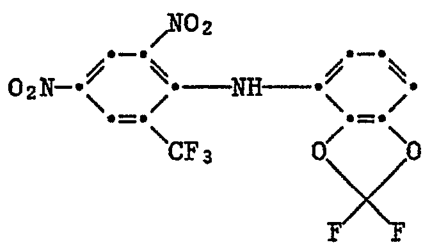

(I).

2. A process for the preparation of the compound of formula I, which process comprises reacting the compound of formula II

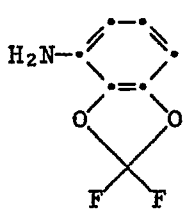

(II)

with the compound of formula III

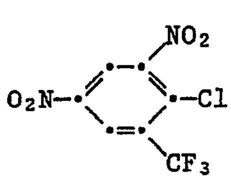

(III)

in the presence of an acid acceptor in an inert solvent.

3. A composition for controlling pests or preventing infestation by pests, which contains as active ingredient the compound of formula I according to claim 1.

4. A composition according to claim 3, which contains 0.1 to 99% of the compound of formula I, 99.9 to 1% of a solid or liquid adjuvant, and 0 to 25% of a surfactant.

5. A composition according to claim 4, which contains 0.1 to 95% of the compound of formula I, 99.8 to 5% of a solid or liquid adjuvant, and 0 to 25% of a surfactant.

6. A process for the preparation of an agrochemical composition according to claim 3, which process comprises homogenously mixing the compound of formula I as defined in claim 1 with suitable solid or liquid adjuvants and surfactants.

7. A method of controlling phytopathogenic pests or of preventing infestation of cultivated plants by such pests, which method comprises applying to said plants or to the locus thereof the compound of formula I as defined in claim 1.

8. Use of the compound of formula I according to claim 1 for controlling microorganisms or for preventing infestation by microorganisms.

9. Use according to claim 8, wherein the microorganisms are phytopathogenic fungi.

10. Use according to claim 9, wherein the phytopathogenic fungi are fungi of the classes Ascomycetes, Basidiomycetes or Fungi imperfecti.

11. Use of the compound of formula I for controlling insects.

12. Use of the compound of formula I for controlling Acarina.

13. The compound of formula II

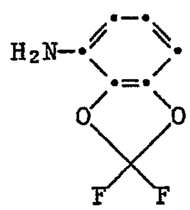

(II)

14. A process for the preparation of the compound of formula II, which process comprises subjecting the compound of formula

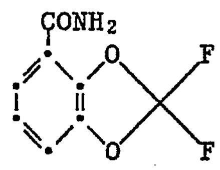

to Hofmann degradation with bromine, in the presence of aqueous alkali metal hydroxide.

**Revendications**

1. 2,2-difluoro-4-(2', 4' -dinitro-6' -trifluorométhyl-anilino)-1,3-benzodioxol de formule I

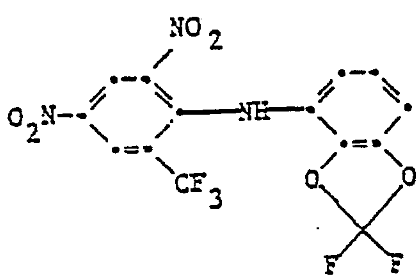

(I).

2. Procédé de préparation du composé de formule I, caractérisé en ce qu'on fait réagir le composé de formule II

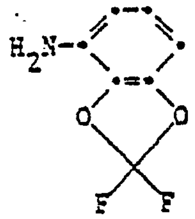

(II)

avec le composé de formule III

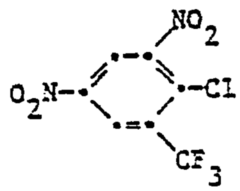

(III)

en présence d'un agent liant d'acide dans un solvant inerte.

3. Agent pour combattre ou prévenir une attaque par des parasites, caractérisé en ce qu'il contient comme composant actif le composé selon la revendication 1.

4. Agent selon la revendication 3, caractérisé en ce qu'il contient de 0,1 à 99% de matière active de formule I, de 99,9 à 1% d'un additif solide ou liquide et de 0 à 25% d'un agent tensio-actif.

5. Agent selon la revendication 4, caractérisé en ce qu'il comprend de 0,1 à 95% de matière active de formule I, de 99,8 à 5% d'un additif solide ou liquide et de 0,1 à 25% d'un agent tensio-actif.

6. Préparation d'un agent agrochimique selon la revendication 3, caractérisé en ce qu'on mélange intimement le composé de formule I défini par la revendication 1 avec des additifs solides ou liquides appropriés et des agents tensio-actifs.

7. Procédé pour combattre ou prévenir une attaque des plantes cultivées par des parasites phytopathogènes, caractérisé en ce qu'on applique le composé de formule I défini dans la revendication 1 sur la plante ou son lieu de croissance.

8. Application du composé de formule I selon la revendication 1 pour combattre et/ou prévenir une attaque de microorganismes.

9. Application selon la revendication 8, caractérisée en ce qu'il s'agit pour les microorganismes de champignons phytopathogènes.

10. Application selon la revendication 9 contre les champignons de la classe des ascomycètes, basidiomycètes ou Fungi imperfecti.

11. Application du composé de formule I pour combattre les insectes.

12. Application du composé de formule I pour combattre les acariens.

13. Composé de formule II

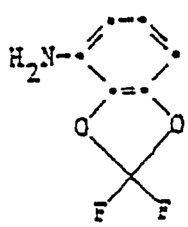

(II)

14. Procédé de préparation du composé de formule II, caractérisé en ce qu'on soumet le composé de formule

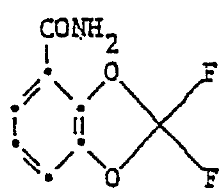

avec du brome en présence d'un hydroxyde alcalin aqueux à la dégradation de Hofmann.